# EUROPEAN PATENT APPLICATION

(11) **EP 3 919 075 A2**
(43) Date of publication of application: **08.12.2021**
(21) Application number: 20748794.3
(22) Date of filing: 23.01.2020
(51) Int. Cl.: A61K 39/04, A61P 31/06, C07K 14/35, C12N 15/82, A61K 39/00

(54) **VACCINE COMPOSITION FOR PREVENTING TUBERCULOSIS COMPRISING GLYCOSYLATED AG85A PROTEIN AND METHOD FOR PREPARING SAME**

(30) Priority: 28.01.2019 KR 20190010431
(71) Applicant: Bioapplications Inc., Pohang-si, Gyeongsangbuk-do 37668 (KR); Industry-Academic Cooperation Foundation, Yonsei University, Seoul 03722 (KR); Quratis Inc., Seoul 08375 (KR)
(72) Inventor: LEE, Yong Jik, Pohang-Si, Gyeongsangbuk-do 37668 (KR); SOHN, Eun-Ju, Pohang-Si, Gyeongsangbuk-do 37668 (KR); SHIN, Sung Jae, Seoul 03722 (KR); KIM, Hongmin, Seoul 03722 (KR); CHO, Kwan Goo, Seoul 08375 (KR); OH, Myung Ryurl, Seoul 08375 (KR)
(74) Representative: Dehns
(86) International application number: PCT/KR2020/001230
(87) International publication number: WO 2020/159169

(57) **Abstract**

The present invention relates to a vaccine composition for preventing tuberculosis comprising a glycosylated Ag85A protein, a vector for preparing the protein, a transformant using the vector, and a method for producing the glycosylated Ag85A protein by using the transformant. A vaccine composition comprising a glycosylated Ag85A protein of the present invention has the effect of inducing an increase in multifunctional T cells simultaneously secreting IFN-γ, TNF-α, and IL-2 which are important in regard to a protective effect against tuberculosis, and thus can be usefully used as a vaccine for preventing tuberculosis. Furthermore, the glycosylated Ag85A protein can be effectively expressed in plants and separated with high yield by means of a vector optimized for protein production, and thus can be mass produced at low cost.

## Description

### [Technical Field]

The present invention relates to a vaccine composition for preventing tuberculosis comprising a glycosylated Ag85A protein, a vector for preparing the protein, a transformant using the vector, and a method for producing the glycosylated Ag85A protein by using the transformant.

This application claims priority to and the benefit of Korean Patent Application No. 10-2019-0010431 filed in the Korean Intellectual Property Office on January 28, 2019, and all the contents disclosed in the specification and drawings of that application are incorporated in this application.

### [Background Art]

Tuberculosis is one of the three major infectious diseases managed by the World Health Organization (WHO), was discovered by a microbiologist Robert Koch in 1882, and is a fatal disease which is caused by *Mycobacerium tuberculosis* and exhibits high incidence and mortality rates. About 60 million patients worldwide have been infected with active tuberculosis, about 50 million to 100 million are estimated to be newly infected with tuberculosis each year, at least 9 million new cases of tuberculosis occur each year, and 1.5 million are known to die from tuberculosis annually. The tuberculosis incidence rate is 146 per 100 thousand people, and the tuberculosis mortality rate is 49 per 100 thousand people, accounting for the most common cause of death among single infectious diseases, indicating that tuberculosis remains a serious health problem worldwide.

Meanwhile, in relation to the preparation of a vaccine for preventing tuberculosis as described above, the demand in the medical industry for the mass production of animal proteins is the development of a mixed expression system for producing various complex proteins or peptides. Host organisms include everything from bacteria to eukaryotes such as yeast, insects, mammals and plant cells. However, bacteria can produce relatively large amounts of protein, but form insoluble inclusion bodies in many cases, and have a limitation in a post-translational modification process. In contrast, an eukaryotic cell expression system may also make proteins that are not the same as an originally intended protein due to different post-translational processes, but the system may conjugate sugars to the proteins, and may perform a post-translational process. A plant production system is currently commercially used to synthesize exogenous proteins, and a post-translational modification of plant cells is very similar to that performed in animal cells, allowing a multimeric protein to be accurately produced. In the case of plant-derived animal proteins, amino acid residues that are glycosylated occur in the same manner as in the original protein. However, although the N-linked glycosylation process in the secretory pathway of plant cells shows more diverse forms of glycosylation than those produced in an animal expression system, glycoprotein activity is maintained in many cases.

For vaccines currently attempted to be developed using plants, in the case of pathogenic diseases, proteins that perform an adhesion function essential for bacterial and viral mucosal infections, toxin proteins that show bacterial toxicity, and proteins that make up the viral structure are usually used as antigens, and in the case of non-pathogenic diseases, main enzymes which produce materials that cause the diseases and causative materials are used as antigens. According to the study results conducted to date, since it has been reported that a recombinant antigen protein derived from plants has the same function as animal-derived vaccines, there is a need for various and in-depth studies applying the recombinant antigen protein.

### [Disclosure]

### [Technical Problem]

As a result of performing experiments on an immune response, protective efficacy against tuberculosis, and the like by immunizing mice with a glycosylated Ag85A antigen protein and a non-glycosylated Ag85A antigen protein, the present inventors experimentally confirmed that Ag85A expressed in plant cells and glycosylated has a better protective effect against tuberculosis than non-glycosylated Ag85A, thereby completing the present invention.

Therefore, an object of the present invention is to provide a vaccine composition for preventing tuberculosis, comprising a glycosylated Ag85A protein comprising an amino acid sequence of SEQ ID NO: 1.

However, a technical problem to be achieved by the present invention is not limited to the aforementioned problems, and other problems that are not mentioned may be clearly understood by those skilled in the art from the following description.

### [Technical Solution]

To achieve the object of the present invention as described above, the present invention provides a vaccine composition for preventing tuberculosis, comprising a glycosylated Ag85A protein comprising an amino acid sequence of SEQ ID NO: 1.

As an exemplary embodiment of the present invention, the glycosylated Ag85A protein may induce inhibition of damage to lung tissue infected by *Mycobacterium tuberculosis.*

As another exemplary embodiment of the present invention, the glycosylated Ag85A protein may induce a decrease in the number of *M tuberculosis* in the lungs.

As still another exemplary embodiment of the present invention, the glycosylated Ag85A protein may induce an increase in CD4⁺CD44⁺ T cells simultaneously secreting two or more cytokines selected from the group consisting of IFN-γ (interferon-γ), TNF-α (tumor necrosis factor-α), and IL-2 (interleukin-2).

As yet another exemplary embodiment of the present invention, the glycosylated Ag85A protein may induce an increase in CD8⁺CD44⁺ T cells simultaneously secreting two or more cytokines selected from the group consisting of IFN-γ (interferon-γ), TNF-α (tumor necrosis factor-α), and IL-2 (interleukin-2).

Further, the present invention provides a vector for expressing a vaccine for preventing tuberculosis, comprising: a gene encoding a glycosylated Ag85A protein comprising an amino acid sequence of SEQ ID NO: 1; and a gene encoding cellulose binding module 3 (hereinafter, referred to as CBM3), comprising a base sequence of SEQ ID NO: 2.

In addition, the present invention provides a vector for expressing a vaccine for preventing tuberculosis, comprising: a gene encoding a glycosylated Ag85A protein comprising an amino acid sequence of SEQ ID NO: 1; a gene encoding cellulose binding module 3 (CBM3), comprising a base sequence of SEQ ID NO: 2; and a gene encoding a BiP signal peptide, comprising a base sequence of SEQ ID NO: 3.

Furthermore, the present invention provides a vector for expressing a vaccine for preventing tuberculosis, comprising: a gene encoding a glycosylated Ag85A protein comprising an amino acid sequence of SEQ ID NO: 1; a gene encoding cellulose binding module 3 (CBM3), comprising a base sequence of SEQ ID NO: 2; and a gene encoding an HDEL peptide, comprising a base sequence of SEQ ID NO: 4.

As an exemplary embodiment of the present invention, the vector may further comprise a sequence which is recognized and cleaved by enterokinase, comprising a base sequence of SEQ ID NO: 5.

As another exemplary embodiment of the present invention, the vector may further comprise a gene encoding a linking peptide, comprising a base sequence of SEQ ID: 6.

Further, the present invention provides a transformant for preparing a vaccine for preventing tuberculosis, the transformant transformed with the vector.

As an exemplary embodiment of the present invention, the transformant may be a plant.

As another exemplary embodiment of the present invention, the plant may be one or more dicotyledonous plants selected from the group consisting of Arabidopsis thaliana, soybean, tobacco, eggplant, capsicum, potato, tomato, Chinese cabbage, radish, cabbage, lettuce, peach, pear, strawberry, water melon, Korean melon, cucumber, carrot, and celery; or one or more monocotyledonous plants selected from the group consisting of rice, barley, wheat, rye, corn, sugar cane, oat, and onion.

In addition, the present invention provides a method for producing a vaccine composition for preventing tuberculosis, the method comprising: (a) culturing the transformant; and
(b) isolating and purifying a glycosylated Ag85A protein from the transformant or culture solution.

Furthermore, the present invention provides a method for preventing tuberculosis, the method comprising: administering a vaccine composition comprising a glycosylated Ag85A protein comprising an amino acid sequence of SEQ ID NO: 1 to an individual.

Further, the present invention provides a use of a vaccine composition comprising a glycosylated Ag85A protein comprising an amino acid sequence of SEQ ID NO: 1 for preventing tuberculosis.

In addition, the present invention provides a use of a glycosylated Ag85A protein comprising an amino acid sequence of SEQ ID NO: 1 for preparing a vaccine used for preventing tuberculosis.

### [Advantageous Effects]

A vaccine composition comprising a glycosylated Ag85A protein of the present invention has the effect of inducing an increase in multifunctional T cells simultaneously secreting IFN-γ, TNF-α, and IL-2 which are important for a protective effect against tuberculosis, and thus can be usefully used as a vaccine for preventing tuberculosis. Furthermore, the glycosylated Ag85A protein can be effectively expressed in plants and isolated with high yield by means of a vector optimized for protein production, and thus can be mass produced at low cost.

### [Description of Drawings]

FIG. 1 illustrates the arrangement of genes for expressing a glycosylated Ag85A protein according to an exemplary embodiment of the present invention.
FIG. 2 illustrates the results of performing western blotting in order to confirm the glycosylation of a plant-expressed Ag85A protein.
FIG. 3 illustrates the results of comparing the secretion of IFN-γ after infecting mice with *M. tuberculosis* in order to explore the possibility of a glycosylated Ag85A protein as a vaccine antigen.
FIG. 4 schematically illustrates an experimental method designed for immunological studies of a glycosylated Ag85A protein.
FIGS. 5A to 5C are the results of comparing the proportion of T cells, which secrete one or more cytokines in mice by antigen stimulation, such as an Ag85A protein, four weeks after a final immunization.
FIG. 6A illustrates the results of visual examination and hematoxylin-eosin (H&E) staining of lung tissues after infecting immunized mice with *M tuberculosis.*
FIG. 6B illustrates the results of measuring a colony forming unit (CFU) in the lungs and spleen 4 and 12 weeks after infecting immunized mice with *M*. *tuberculosis.*
FIGS. 7A to 7C illustrate the results of comparing the proportion of T cells, which secrete one or more cytokines, 4 weeks after infecting immunized mice with *M. tuberculosis.*

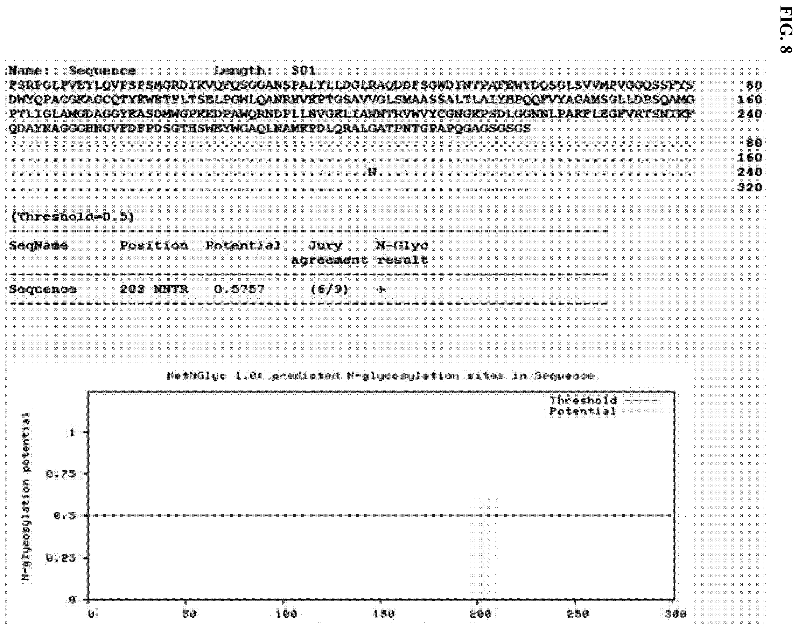

FIG. 8 illustrates the result of operating a program for predicting the possibility of glycosylation using an amino acid sequence of SEQ ID NO: 1 used in the present invention.

### [Modes of the Invention]

As a result of performing experiments on an immune response, protective efficacy against tuberculosis, and the like by immunizing mice with a glycosylated Ag85A antigen protein and a non-glycosylated Ag85A antigen protein, the present inventors experimentally confirmed that Ag85A expressed in plant cells and glycosylated has a better protective effect against tuberculosis than non-glycosylated Ag85A, thereby completing the present invention.

In an exemplary embodiment of the present invention, it was confirmed that glycosylated Ag85A could be produced in plants by isolating and purifying proteins produced using an Ag85A expression vector expressed in plants (see Example 1).

In another exemplary embodiment of the present invention, western blotting was performed to confirm whether an Ag85A protein produced in plants was glycosylated, and as a result, it was confirmed whether a plant-expressed Ag85A protein was glycosylated by confirming glycan cleavage (see Example 2).

In still another exemplary embodiment of the present invention, as a result of measuring the secretion of IFN-γ in lung cells using mice infected with *Mycobacterium tuberculosis,* the possibility of glycosylated Ag85A as a vaccine antigen was explored by confirming the continuous secretion of IFN-γ (see Example 3).

In yet another exemplary embodiment of the present invention, as a result of examining the immunogenicity of a glycosylated Ag85A protein, it was confirmed that in the case of mice immunized with the glycosylated Ag85A protein, the proportion of T cells simultaneously secreting IFN-γ, TNF-α, and IL-2 was increased (see Example 4).

Hereinafter, the present invention will be described in detail.

The present invention provides a glycosylated Ag85A protein comprising an amino acid sequence of SEQ ID NO: 1.

As used herein, the term "Ag85A" is one of the antigens which have been extensively studied, and is known to show effective effects not only as a protein, but also as a DNA vaccine.

As used herein, the term "glycosylation" is divided into N-glycosylation and O-glycosylation as a protein post-translational process of cells (eukaryotes), which vary depending on the functional group to be attached, and a process by which sugars such as lactose are attached to proteins produced in cells is collectively called "glycosylation". When sugar chains are connected to proteins by the glycosylation process, the proteins undergo a "folding" process to form a three-dimensional structure, which imparts stability such that the protein can be maintained for a long time without unfolding. Further, the sugar chain attached to the protein may be transferred to the cell membrane to become a cell membrane protein, thereby exhibiting an antigen-like effect. The glycosylated protein as described above is called a glycoprotein, and examples of a representative glycoprotein include an antibody which plays an important role in an immune response, and the like. As a result of running a program for predicting the possibility of glycosylation using an amino acid sequence of SEQ ID NO: 1 used in the present invention (http://www.cbs.dtu.dk/services/NetNGlyc/), it was predicted that N-glycosylation would occur at asparagine (N) 203 (see Example 8), but is not limited thereto, and modification can be made within a range having the same effect as the present invention.

As another aspect of the present invention, the present invention provides a vaccine composition for preventing tuberculosis, comprising a glycosylated Ag85A protein comprising an amino acid sequence of SEQ ID NO: 1.

As used herein, the "antigen" generally refers to all materials that cause an immune response in the body, and is preferably a virus, a chemical, a bacterium, pollen, a cancer cell, shrimp, and the like or a partial peptide or protein thereof, but is not limited thereto as long as it is a material that may cause an immune response in the body.

As used herein, the term "vaccine" is a biological preparation containing an antigen that causes an immune response in an organism, and refers to an immunogen that induces immunity in an organism by injection or oral administration into a human or animal for prevention of an infectious disease. The animal is a human or non-human animal, and the non-human animal refers to a pig, a cow, a horse, a dog, a goat, sheep, and the like, but is not limited thereto.

As used herein the term "vaccine composition" may be used by being formulated in the form of an oral formulation such as a powder, granules, a tablet, a capsule, a suspension, an emulsion, a syrup, and an aerosol, and a sterile injection solution, according to a typical method. When the composition is prepared, the composition may be prepared using a commonly used diluent or excipient, such as a filler, an extender, a binder, a wetting agent, a disintegrant, and a surfactant. A solid formulation for oral formulation includes a tablet, a pill, a powder, a granule, a capsule, and the like, and the solid formulation may be prepared by mixing at least one excipient, for example, starch, calcium carbonate, sucrose or lactose, gelatin, and the like with a lecithin-like emulsifier. Further, in addition to simple excipients, lubricants such as magnesium stearate and talc may also be used. As a liquid formulation for oral administration, a suspension, a liquid for internal use, an emulsion, a syrup, and the like may be used, and in addition to water and liquid paraffin which are simple commonly used diluents, various excipients, for example, a wetting agent, a sweetener, an aromatic, a preservative, and the like, may be included. Examples of a formulation for parenteral administration include an aqueous sterile solution, a non-aqueous solvent, a suspension, an emulsion, and a freeze-dried preparation. As the non-aqueous solvent and the suspension, it is possible to use propylene glycol, polyethylene glycol, a vegetable oil such as olive oil, an injectable ester such as ethyl oleate, and the like. Furthermore, an "immune antigen adjuvant" conventionally known may be further included. The adjuvant may be used without limitation as long as it is known in the art, but for example, immunogenicity may be enhanced by further including Freund's Complete Adjuvant or Incomplete Adjuvant.

The vaccine composition or pharmaceutical composition of the present invention may be used by being formulated in the form of an oral formulation such as powder, granules, a tablet, a capsule, a suspension, an emulsion, a syrup, and an aerosol, and an external preparation, a suppository, or a sterile injection solution, according to a typical method.

The route of administration of the vaccine composition according to the present invention includes, but is not limited to, oral, intravenous, intramuscular, intraarterial, intramedullary, intrathecal, intracardiac, transdermal, subcutaneous, intraperitoneal, intranasal, intestinal, topical, sublingual or rectal routes. Oral or parenteral administration is preferred. As used herein, the term "parenteral" includes subcutaneous, intradermal, intravenous, intramuscular, intraarticular, intrasynovial, intrasternal, intrathecal, intralesional and intracranial injection or infusion techniques. The vaccine composition of the present invention may also be administered in the form of a suppository for rectal administration.

The dose of the vaccine composition or pharmaceutical composition according to the present invention is selected in consideration of the age, body weight, sex, physical condition and the like of an individual. The amount required to induce an immunoprotective response in an individual without particular side effects may vary depending on the recombinant protein used as an immunogen and the presence of a random excipient. In general, each dose contains 0.1 to 1000 µg, preferably 0.1 to 100 µg of a protein per ml of a sterile solution of the recombinant protein of the present invention. In the case of the vaccine composition, an initial dose followed by optionally repeated antigenic stimulation may be performed, if necessary.

As used herein, the "immune antigen adjuvant" generally refers to any material that increases the humoral and/or cell-mediated immune response to an antigen.

As used herein, the "prevention" refers to all actions that inhibit tuberculosis or delay the onset of tuberculosis by administering the glycosylated Ag85A protein according to the present invention.

In the present invention, "tuberculosis" includes ocular tuberculosis, cutaneous tuberculosis, adrenal tuberculosis, renal tuberculosis, tuberculosis of epididymis, lymphatic gland tuberculosis, laryngeal tuberculosis, tuberculous otitis media, intestinal tuberculosis, multidrug-resistant tuberculosis, pulmonary tuberculosis, biliary tuberculosis, bone tuberculosis, throat tuberculosis, lymphatic tuberculosis, lung deficiency pattern, breast tuberculosis and spinal tuberculosis, and is not limited thereto.

In the present invention, "inhibition" is meant to include prevention in advance, alleviation, or treatment of tuberculosis by administering the glycosylated Ag85A protein according to the present invention.

As another aspect of the present invention, the present invention provides a method for preventing tuberculosis, the method comprising: administering a vaccine composition comprising a glycosylated Ag85A protein comprising an amino acid sequence of SEQ ID NO: 1 to an individual.

As still another aspect of the present invention, the present invention provides a use of a vaccine composition comprising a glycosylated Ag85A protein comprising an amino acid sequence of SEQ ID NO: 1 for preventing tuberculosis.

As yet another exemplary embodiment of the present invention, the present invention provides a use of a glycosylated Ag85A protein comprising an amino acid sequence of SEQ ID NO: 1 for preparing a vaccine used for preventing tuberculosis.

As used herein, the "individual" refers to a subject to which the glycosylated Ag85A protein of the present invention may be administered, and the subject is not limited.

As used herein, the "administration" refers to the provision of a predetermined vaccine composition of the present invention to an individual by any appropriate method.

In the present invention, the glycosylated Ag85A protein may induce inhibition of lung tissue damage caused by *Mycobacterium tuberculosis* infection.

In the present invention, the glycosylated Ag85A protein may induce a decrease in the number of *Mycobacterium tuberculosis* in the lungs.

In the present invention, the glycosylated Ag85A protein may induce an increase in multifunctional T cells simultaneously secreting two or more cytokines selected from the group consisting of IFN-γ (interferon-γ), TNF-α (tumor necrosis factor-α), and IL-2 (interleukin-2), and the T cells may be CD4⁺CD44⁺ T cells, or CD8⁺CD44⁺ T cells, but are not limited thereto.

As yet another aspect of the present invention, the present invention provides a vector for expressing a vaccine for preventing tuberculosis, comprising: a gene encoding a glycosylated Ag85A protein comprising an amino acid sequence of SEQ ID NO: 1; and a gene encoding cellulose binding module 3 (CBM3), comprising a base sequence of SEQ ID NO: 2.

As used herein, the "expression vector" refers to a vector capable of expressing a peptide or protein encoded by a foreign nucleic acid inserted in the vector, preferably a vector prepared so as to express a glycosylated Ag85A protein. The "vector" refers to any medium for the introduction and/or transfer of a base into a host cell in vitro, ex vivo, or in vivo, and may be a replicon to which another DNA fragment may be bound to bring about the replication of the bound fragment, and the "replicon" refers to any genetic unit (for example, a plasmid, a phage, a cosmid, a chromosome, a virus, and the like) that functions as an autonomous unit of DNA replication in vivo, that is, one which is capable of replication under its own control. A recombinant expression vector of the present invention may preferably include a promoter, which is a transcription initiation factor to which RNA polymerase binds, any operator sequence for regulating transcription, a sequence encoding a suitable mRNA ribosome binding site and a sequence regulating the termination of transcription and translation, a terminator, and the like, may more preferably further include a 5' UTR site gene of M17 for increasing the synthesis amount of a protein, and the promoter may include, for example, a pEMU promoter, a MAS promoter, a histone promoter, a Clp promoter, a 35S promoter derived from a cauliflower mosaic virus, a 19S RNA promoter derived from a cauliflower mosaic virus, an actin protein promoter of a plant, an ubiquitin protein promoter, a cytomegalovirus (CMV) promoter, a simian virus 40 (SV40) promoter, a respiratory syncytial virus (RSV) promoter, an elongation factor-1 alpha (EF-1α) promoter, and the like, an example of the terminator is nopaline synthase (NOS), a rice α-amylase RAmy1 A terminator, a phaseoline terminator, a terminator for the optopine gene of *Agrobacterium tumefaciens,* a rrnB1B2 terminator of *E. coli,* and the like, and the above examples are merely exemplary and are not limited thereto.

In addition, the present invention provides a vector for expressing a vaccine for preventing tuberculosis, comprising: a gene encoding a glycosylated Ag85A protein comprising an amino acid sequence of SEQ ID NO: 1; a gene encoding cellulose binding module 3 (CBM3), comprising a base sequence of SEQ ID NO: 2; and a gene encoding a BiP signal peptide, comprising a base sequence of SEQ ID NO: 3.

Furthermore, the present invention provides a vector for expressing a vaccine for preventing tuberculosis, comprising: a gene encoding a glycosylated Ag85A protein comprising an amino acid sequence of SEQ ID NO: 1; a gene encoding cellulose binding module 3 (CBM3), comprising a base sequence of SEQ ID NO: 2; and a gene encoding an HDEL peptide, comprising a base sequence of SEQ ID NO: 4.

In the present invention, the vector may further comprise a sequence which is recognized and cleaved by enterokinase, comprising a base sequence of SEQ ID NO: 5.

In the present invention, the vector may further comprise a gene encoding a linking peptide, comprising a base sequence of SEQ ID: 6.

In the present invention, the vector is constructed by sequentially linking a gene encoding CBM3, a gene encoding a linking peptide, an enterokinase cleavage site, and a gene encoding an Ag85A protein, a gene encoding a BiP signal peptide capable of targeting a target protein in a plant cell to the endoplasmic reticulum is linked to the 3' end of CBM3, and a gene encoding His-Asp-Glu-Leu (HDEL) allowing a vector to be retained in the endoplasmic reticulum may be linked to the carboxyl end of a gene encoding the target protein, but is not limited thereto.

As yet another aspect of the present invention, the present invention provides a transformant for preparing a vaccine for preventing tuberculosis, the transformant transformed with the glycosylated Ag85A protein expression vector.

As used herein, the "transformation" collectively refers to changes in genetic properties of a living organism by injected DNA, the "transgenic organism" is an organism prepared by injecting an external gene by a molecular genetic method, preferably, an organism transformed by a recombinant expression vector of the present invention, and the organism is not limited as long as it is a living organism such as a microorganism, a eukaryotic cell, an insect, an animal, and a plant, and is preferably Escherichia coli, Salmonella, Bacillus, yeast, an animal cell, a mouse, a rat, a dog, a monkey, a pig, a horse, a cow, Agrobacterium tumefaciens, a plant, and the like, but is not limited thereto. The transformant may be prepared by a method such as transformation, transfection, Agrobacterium-mediated transformation, particle gun bombardment, sonication, electroporation, and polyethylene glycol (PEG)-mediated transformation, but there is no limitation as long as it is a method capable of injecting the vector of the present invention.

In the present invention, the transformant is preferably a plant. The production of a useful material from plants has many benefits, and has advantages in that 1) a production unit price can be significantly reduced, 2) various sources of contamination (viruses, oncogenes, enterotoxins, and the like) that can be generated from conventional popular methods (synthesizing in animal cells and microorganisms to separate and purify proteins) can be fundamentally eliminated, and 3) seed stock can be managed unlike animal cells or microorganisms, even at a commercialization stage. Further, there is also an advantage in that 4) it is possible to supply the corresponding material according to the increased demand in the shortest period of time because it is absolutely advantageous compared to the existing animal cell system in terms of installation technology and costs required for mass production when the demand for the material increases rapidly. The background that a method of producing a useful material by transforming a plant as described above is attracting attention lies in a protein synthesis pathway of the plant. As the post-translational modification process is essential for mammalian protein synthesis, since plants have a eukaryotic protein synthesis pathway, it is possible to produce proteins which are almost similar to proteins expressed in mammals.

In the present invention, the plant may be one or more dicotyledonous plants selected from the group consisting of Arabidopsis thaliana, soybean, tobacco, eggplant, capsicum, potato, tomato, Chinese cabbage, radish, cabbage, lettuce, peach, pear, strawberry, water melon, Korean melon, cucumber, carrot, and celery; or one or more monocotyledonous plants selected from the group consisting of rice, barley, wheat, rye, corn, sugar cane, oat, and onion, but is not limited thereto.

As yet another aspect of the present invention, the present invention provides a method for producing a vaccine composition for preventing tuberculosis, the method comprising: (a) culturing the transformant; and
(b) isolating and purifying a glycosylated Ag85A protein from the transformant or culture solution.

Meanwhile, Table 1 below summarizes sequences used in the present invention.

**[Table 1]**

| SEQ ID NO | Sequence |
|---|---|
| 1 (amino acid sequence of Ag85A protein) | FSRPGLPVEYLQVPSPSMGRDIKVQFQSGGANSPALYLLDGLR AQDDFSGWDINTPAFEWYDQSGLSVVMPVGGQSSFYSDWY QPACGKAGCQTYKWETFLTSELPGWLQANRHVKPTGSAVVG LSMAASSALTLAIYHPQQFVYAGAMSGLLDPSQAMGPTLIGL AMGDAGGYKASDMWGPKEDPAWQRNDPLLNVGKLIANNTR VWVYCGNGKPSDLGGNNLPAKFLEGFVRTSNIKFQDAYNAG GGHNGVFDFPDSGTHSWEYWGAQLNAMKPDLQRALGATPN TGPAPQGAGSGSGS |
| 2 (base sequence of CBM3) | GTATCAGGTAACCTTAAGGTGGAGTTTTACAACTCGAACCC TTCTGATACAACTAACTCAATAAACCCACAGTTCAAAGTTA CAAACACAGGCAGCTCTGCGATCGATTTGTCTAAATTAACC CTCAGATACTATTATACGGTTGATGGACAGAAGGACCAGAC TTTCTGGTGTGATCATGCAGCTATCATTGGTTCTAACGGTAG CTACAACGGAATTACATCAAACGTGAAGGGCACTTTCGTTA AGATGTCCTCTAGCACTAACAACGCAGACACATATTTGGAG ATCAGTTTTACGGGGGGAACCCTTGAACCAGGTGCTCACG TCCAGATTCAAGGAAGATTCGCTAAAAACGACTGGTCGAA CTATACCCAAAGTAATGATTACAGTTTTAAATCCGCCTCACA ATTTGTTGAGTGGGATCAGGTCACTGCTTACCTGAACGGGG TTCTAGTGTGGGGAAAGGAACCTGGT |
| 3 (base sequence of BiP) | ATGGCTCGCTCGTTTGGAGCTAACAGTACCGTTGTGTTGGC GATCATCTTCTTCGGTGAGTGATTTTCCGATCTTCTTCTCCG ATTTAGATCTCCTCTACATTGTTGCTTAATCTCAGAACCTTTT TTCGTTGTTCCTGGATCTGAATGTGTTTGTTTGCAATTTCAC GATCTTAAAAGGTTAGATCTCGATTGGTATTGACGATTGGAA TCTTTACGATTTCAGGATGTTTATTTGCGTTGTCCTCTGCAAT AGAAGAGGCTACGAAGTTA |
| 4 (base sequence of HDEL) | CACGATGAGCTC |
| 5 (base sequence recognized and cleaved by enterokinase) | GATGACGACGATAAA |
| 6 (base sequence of linking peptide) | GAGGCAGCCGCTAAGGAAGCTGCAGCGAAAGCC |
| 7 (base sequence of Ag85A) | TTTAGCCGGCCTGGCCTGCCTGTGGAATACCTGCAGGTGCC TAGCCCTAGCATGGGCCGGGACATCAAAGTGCAGTTTCAG AGCGGCGGGGCTAATAGCCCTGCTCTGTACCTGCTCGATGG CCTGCGGGCTCAGGATGATTTAGCGGCTGGGACATCAACA CCCCTGCTTTTGAATGGTACGATCAGAGCGGCCTGAGCGTC GTGATGCCTGTGGGCGGGCAGAGCAGCTTCTACAGCGATT GGTATCAGCCTGCTTGCGGCAAAGCTGGCTGCCAGACCTA CAAGTGGGAAACCTTTCTGACCAGCGAACTGCCTGGCTGG CTGCAGGCTAATCGGCACGTGAAACCTACCGGCAGCGCCG TGGTGGGCCTGAGCATGGCTGCTAGCTCCGCTCTGACCCTG GCTATCTACCACCCTCAGCAGTTTGTGTACGCTGGCGCTAT GAGCGGCCTGCTCGATCCCTCCCAGGCTATGGGCCCTACCC TGATCGGGCTCGCTATGGGGGATGCTGGGGGGTACAAAGCT AGCGATATGTGGGGCCCTAAAGAAGATCCTGCTTGGCAGC GGAATGATCCTCTGCTCAACGTGGGCAAACTGATCGCTAAT AATACCCGAGTGTGGGTGTACTGCGGCAATGGCAAACCTA GCGATCTGGGCGGGAACAATCTGCCTGCTAAATTTCTGGAA GGCTTCGTGCGGACCAGCAACATCAAGTTTCAGGATGCTTA CAATGCTGGCGGGGGCCACAATGGCGTATTTGATTTTCCTG ATAGCGGCACCCACAGCTGGGAATACTGGGGCGCTCAGCT GAATGCTATGAAACCTGATCTGCAGCGGGCTCTGGGCGCTA CCCCTAATACCGGCCCTGCTCCTCAGGGCGCTGGCTCCGGA TCTGGTAGT |
| 8 (amino acid sequence of CBM3) | VSGNLKVEFYNSNPSDTTNSINPQFKVTNTGSSAIDLSKLTLR YYYTVDGQKDQTFWCDHAAIIGSNGSYNGITSNVKGTFVKM SSSTNNADTYLEISFTGGTLEPGAHVQIQGRFAKNDWSNYTQ SNDYSFKSASQFVEWDQVTAYLNGVLVWGKEPG |

Hereinafter, preferred Examples for helping the understanding of the present invention will be suggested. However, the following Examples are provided for easier understanding of the present invention, and the contents of the present invention are not limited by the following Examples.

### [Examples]

### Example 1. Construction of plant expression A₂85A expression vector and isolation and purification of protein

### 1-1. Construction of CBM3 fusion Ag85A plant expression vector

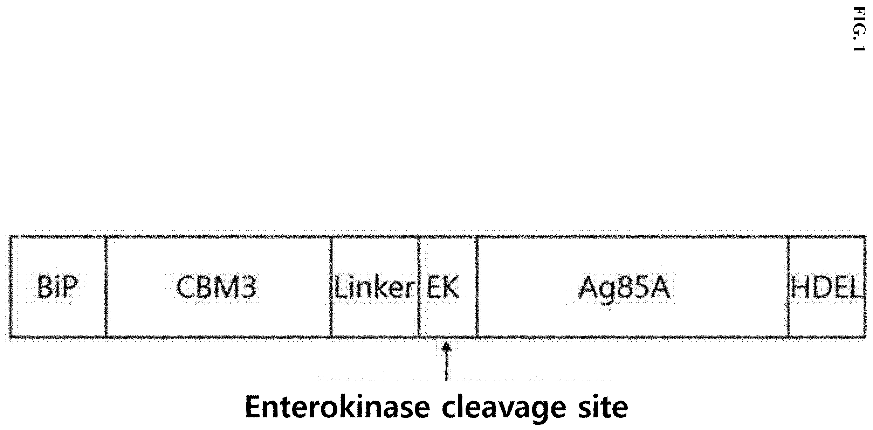

A recombinant plant expression vector was constructed so as to express a CBM3 fusion Ag85A in plants. More specifically, the BiP signal peptide was used to allow a target protein to be moved to the endoplasmic reticulum such that the CBM3 fusion Ag85A protein could be moved to the endoplasmic reticulum, and His-Asp-Glu-Leu (HDEL) was bound to the carboxyl end such that the fusion protein could be accumulated and stored in the endoplasmic reticulum. CBM3 required for the isolation of a fusion protein, a sequence encoding a linking peptide, and a sequence which is recognized and cleaved by enterokinase were bound in front of a gene encoding Ag85A, and then inserted into a plant expression vector pCAMBIA 1300, thereby constructing a recombinant vector (see FIG. 1).

### 1-2. Transient expression of plant expression vector

An Agrobacterium LBA4404 strain was transformed with the plant expression vectors prepared in Example 1-1 using an electric shock method electroporation. After the transformed agrobacteria were shake-cultured in 5 mL of a YEP liquid medium (10 g of yeast extract, 10 g of peptone, 5 g of NaCl, 50 mg/L canamycin, and 25 mg/L rifampicin) under the condition of 28°C for 16 hours, 1 ml of a primary culture medium was inoculated into 50 ml of a fresh YEP medium and shake-cultured under the condition of 28°C for 6 hours.

The agrobacteria cultured as described above were collected by centrifugation (7,000 rpm, 4°C, 5 minutes), and then suspended again in an infiltration buffer (10 mM MES (pH 5.7), 10 mM MgCl₂, and 200 µM acetosyringone) at a concentration of O.D. 1.0 at a wavelength of 600 nm. Agro-infiltration was performed by a method of injecting the agrobacterial suspension into the backside of leaves of Nicotiana benthamiana using a syringe from which the injection needle had been removed.

### 1-3. Isolation and purification of Ag85A

50 mL of a protein extraction buffer solution (50 mM Tris (pH 7.2), 150 mM NaCl, 0.1% Triton X-100, and a 1 X protease inhibitor) was added to 10 g of the Nicotiana benthamiana prepared in Example 1-2, tissue was crushed by a blender, and then centrifuged at 13,000 rpm and 4°C for 20 minutes to recover a protein extract. After 10 g of hydrated microcrystalline cellulose was added to the protein extract, the resulting mixture was mixed well at 4°C for 1 hour to allow the CBM3 fusion Ag85A protein to bind to the microcrystalline cellulose. The microcrystalline cellulose to which the CBM3 fusion Ag85A protein was bound was collected by centrifugation at 13,000 rpm and 4°C for 10 minutes, and then washed twice with 50 mL of a wash buffer solution (50 mM Tris (pH 7.2), 150 mM NaCl) and suspended again in 10 mL of an enterokinase reaction solution (50 mM Tris (pH 7.2), 150 mM NaCl, and 1 mM CaCl₂). As much as 20 units of enterokinase were added to the suspension, the resulting mixture was reacted at 28°C for 4 hours, and then the reaction solution comprising enterokinase and Ag85A was separated from cellulose by centrifugation (14,000 rpm, 4°C, 10 minutes). Then, in order to remove enterokinase from the reaction solution, STI-Sepharose was added to the reaction solution, the resultant was reacted at 4°C for 1 hour, and then isolated and purified Ag85A not bound to STI-Sepharose was obtained by centrifugation (14,000 rpm, 4°C, and 10 minutes).

### Example 2. Confirmation of glycosylation of plant-expressed Ag85A protein

Endo-H (NEB, Cat. No. P0702S) and PNGase F (NEB, Cat. No. P0704S) were used to confirm whether Ag85A was glycosylated. For Endo-H treatment, 1 ug of the isolated and purified Ag85A protein was mixed with 1 uL of a 10 X (multiple) glycoprotein denaturing buffer (5% SDS, 400 mM DTT) and filled with distilled water to prepare a reaction solution having a final volume of 10 uL. The reaction solution was placed in boiling water for 10 minutes for denaturation, then placed on ice for a while to cool lightly, and then 2 uL of 10 X Glycobuffer 3 (500 mM sodium acetate, pH 6), 1 uL of Endo-H enzyme and 7 uL of distilled water were each added to the reaction solution and reacted at 37°C for 1 hour. Treatment of PNGase F was performed in the same manner as Endo-H in the denaturing process, and a reaction solution having a final volume of 20 uL was prepared by adding 2 uL of 10 X Glycobuffer 2 (500 mM sodium phosphate, pH 7.5), 2 µL of 10% NP-40, 1 µL of PNGase F enzyme, and 5 µL of distilled water thereto, and reacted at 37°C for 1 hour. A completely reacted protein and an untreated protein were simultaneously subjected to western blotting, and in this time, an Ag85A antibody (Abcam, Cat. No. ab 193499) was used as an antibody.

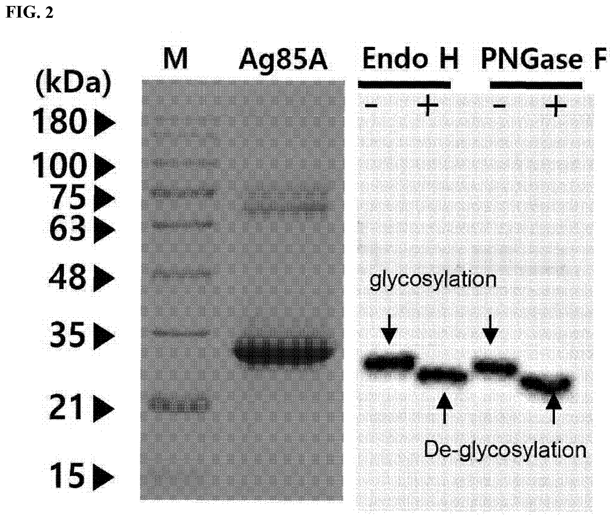

As a result, as illustrated in FIG. 2, it was confirmed that in the case of using both Endo-H enzyme and PNGase F enzyme, a de-glycosylation band appeared when western blotting was performed, indicating glycans were cleaved.

### Example 3. Verification of potential of G-Ag85A as tuberculosis antigen

The degree of IFN-γ secretion was investigated to know whether the corresponding antigen acted as an antigen during actual infection with *M*. *tuberculosis* using mouse lung cells infected with *M. tuberculosis* and whether it could be used as a vaccine candidate material. First, mice were infected with 200 colony forming units (CFUs) of Beijing strain HN878 *M. tuberculosis* via an aerosol route, lung cells (5X10⁵ cells) were isolated by sacrificing 6 mice along with a non-infected control, respectively, 4 weeks or 12 weeks later, non-glycosylated Ag85A (hereinafter, referred to as NG-Ag85A) produced in *E. coli* and glycosylated Ag85A (hereinafter, referred to as G-Ag85A) produced in plants were reacted, respectively at a concentration of 5 ug/ml and 37°C for 12 hours, and then secreted interferon-gamma in a supernatant of the reaction solution was measured by an ELISA kit.

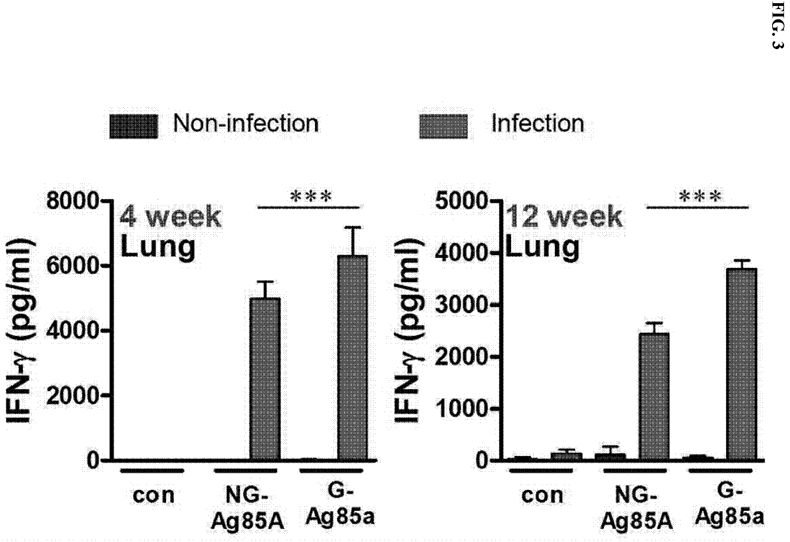

As a result, as illustrated in FIG. 3, it was confirmed that plant-expressed Ag85A (G-Ag85A) acts on mouse lung cells 4 weeks or 12 weeks after infection with *M. tuberculosis* to continuously secrete IFN-γ, and the amount thereof was increased to a significant level compared to *E*. coli-expressed Ag85A (NG-Ag85A). This means that Ag85A is an antigen having immunogenicity against *M. tuberculosis* infection, and possibility of G-Ag85A along with NG-Ag85A as a vaccine antigen was explored.

### Example 4. Investigation of immunogenicity of G-Ag85A

### 4-1. Immunogenicity experimental design

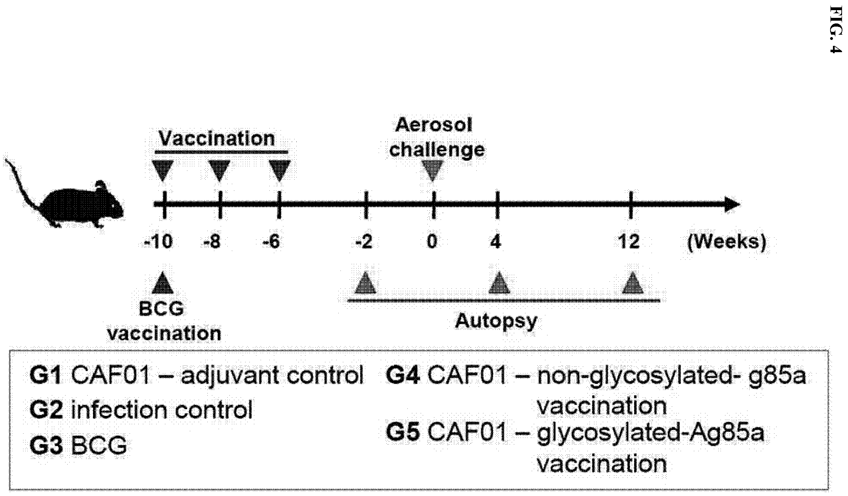

In order to investigate the immunogenicity of a plant-expressed glycosylated tuberculosis antigen, G-Ag85A, mice were immunized three times with BCG, NG-Ag85A, and G-Ag85A at intervals of 2 weeks. Immune responses and protective efficacies were investigated by sacrificing mice 4 weeks (2 weeks before challenge inoculation of *M. tuberculosis)* after a final immunization and 4 weeks and 12 weeks after the challenge inoculation, respectively (see FIG. 4).

### 4-2. Post-immunity antigen-specific multifunctional T cell induction

Multifunctional T cells are known as one of the important host components to defend against *M. tuberculosis,* and are T cells which secrete all of IFN-γ, TNF-α, and IL-2. In this regard, lung cells (5 X 10⁵ cells) were isolated by sacrificing 6 mice of each group 4 weeks after the final immunization and reacted with the NG-Ag85A or G-Ag85A antigen protein at a concentration of 5 ug/ml and 37°C for 12 hours. After the reaction, CD4⁺CD62L⁻ and CD8⁺CD62L⁻ T cells secreting IFN-γ, TNF-α, and/or IL-2 were analyzed by cytometry, and in this case, the gating results for CD4⁺ and CD8⁺ were confirmed.

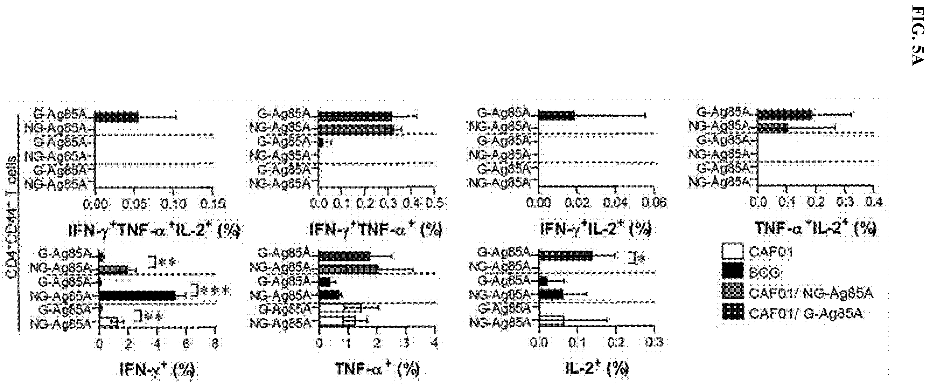

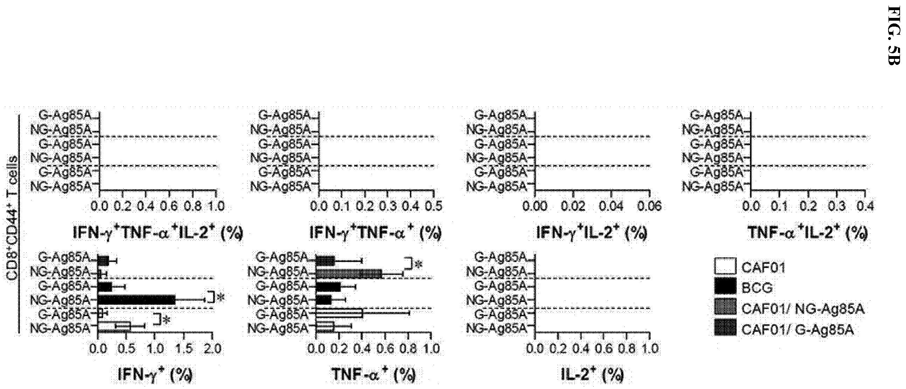

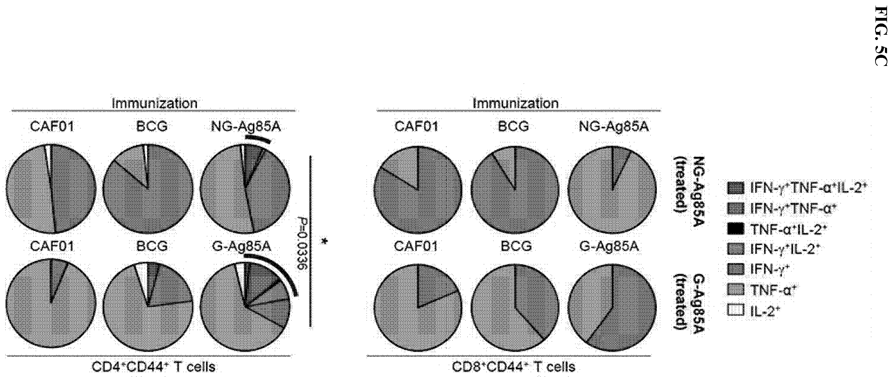

As a result, as illustrated in FIGS. 5A to 5C, it was confirmed that in the case of immunization with G-Ag85A, the poly-cytokine secretion proportion of CD4⁺CD44⁺ T cells increased to a significant level compared to that of the NG-Ag85A group.

### 4-3. Protective efficacy of plant-expressed antigen G-Ag85A against tuberculosis

After mice were infected with 200 CFUs of HN878 *M. tuberculosis* 4 weeks after the final immunization, lung tissue lesions were visually examined by sacrificing the mice 4 and 12 weeks later, and colony forming units (CFUs) in lung and spleen cells were examined.

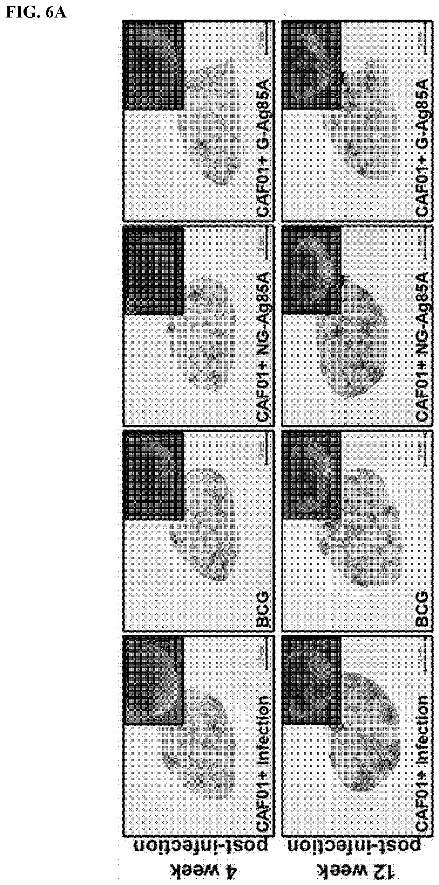

As a result, as illustrated in FIG. 6A, it was confirmed that even by visual findings, the degree of damage to lung tissue of the G-Ag85A group was minor compared to the BCG or NG-Ag85A group, it was confirmed that even in observation by a hematoxylin-eosin (H&E) staining method, the degree of damage to tissue of the G-Ag85A group was milder compared to the BCG or NG-Ag85A group, and in particular, it was confirmed that at 12 weeks after infection, the difference in effect was exhibited enough to confirm the protective effect by the naked eye.

Further, lung and spleen tissues were crushed and cultured, and the bacteria were calculated as log10CFU while being enumerated and used for statistical analysis.

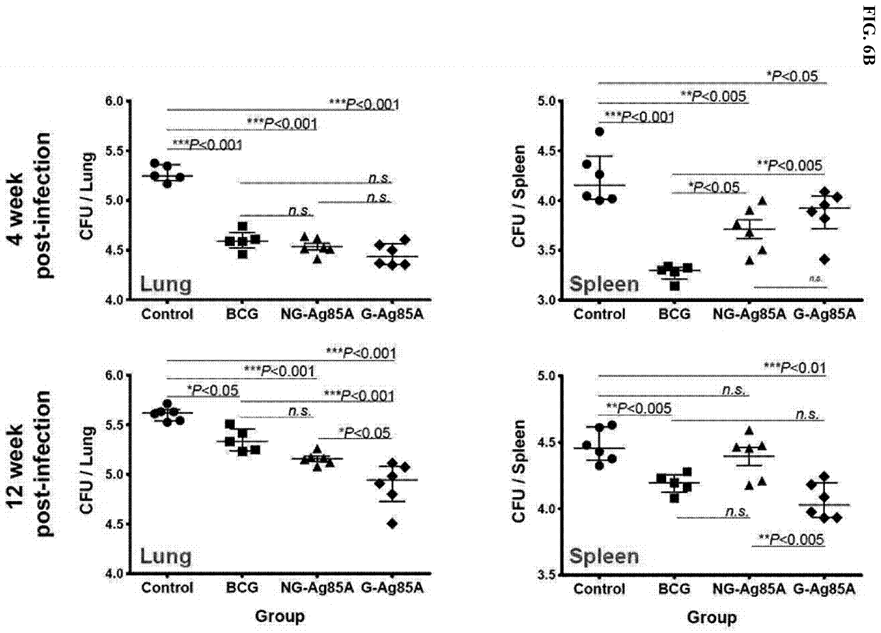

As a result, as illustrated in FIG. 6B, it was confirmed that in the lungs, CFUs were significantly decreased in all of BCG, NG-Ag85A, and G-Ag85A compared to a non-inoculation group 4 weeks after infection, and it was confirmed that in the spleen, all of the three types of inoculation groups also exhibited a decrease in CFUs compared to the non-inoculation group. In the lungs 12 weeks after infection, the G-Ag85A inoculation group showed the lowest CFUs, so that it was confirmed that the G-Ag85A inoculation group had the highest protective effect, and it was confirmed that in the spleen, the G-Ag85A group also showed a better effect than the NG-Ag85A group.

### 4-4. Antigen-specific multifunctional T cell induction at 4 weeks after infection

Lung cells (5 X 10⁵ cells) were isolated by sacrificing 6 mice of each group 4 weeks after infection when the T cell response for inhibition of infection is established in lung tissue of mice infected with *M tuberculosis* and reacted with the NG-Ag85A or G-Ag85A antigen protein at a concentration of 5 ug/ml and 37°C for 12 hours. After the reaction, CD4⁺CD62L⁻ and CD8⁺CD62L⁻ T cells secreting IFN-γ, TNF-α, and/or IL-2 were analyzed by cytometry, and in this case, the gating results for CD4⁺ and CD8⁺ were confirmed.

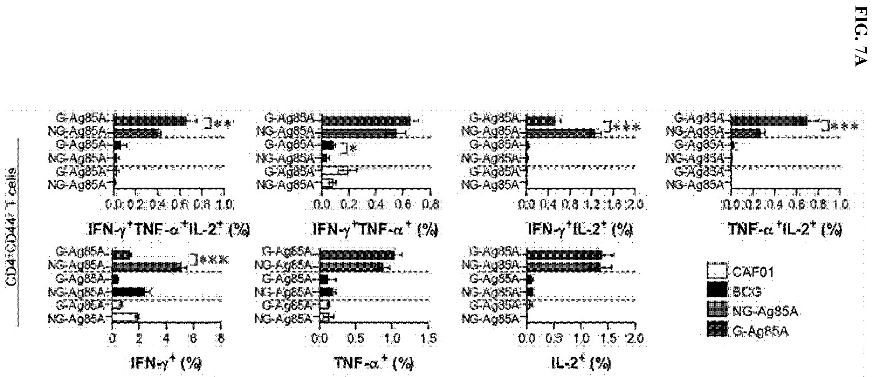

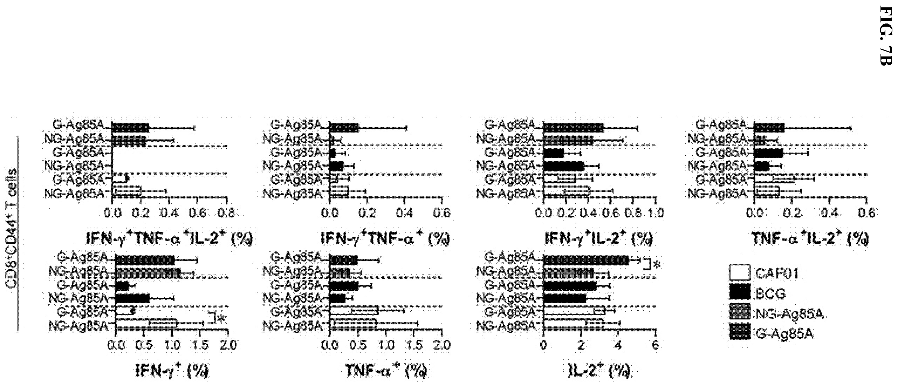

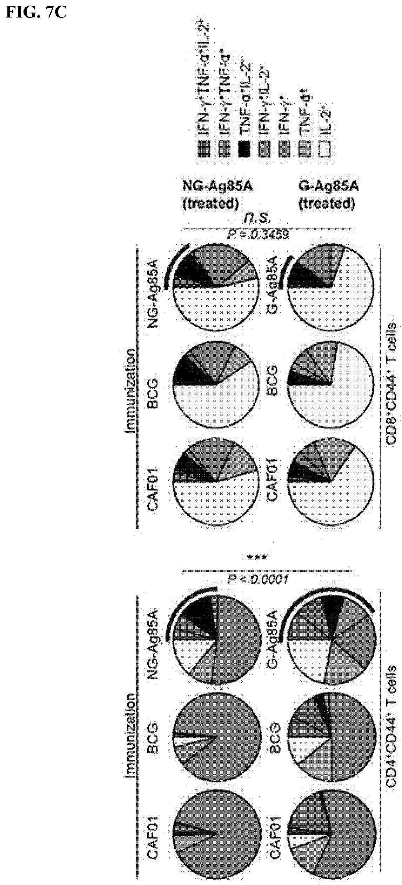

As a result, as illustrated in FIGS. 7A to 7C, it was confirmed that in the case of immunization with G-Ag85A, the poly-cytokine secretion proportion of CD4⁺CD44⁺ T cells increased to a significant level compared to that of the NG-Ag85A group, and in particular, it was confirmed that CD4⁺CD44⁺ multifunctional T cells simultaneously secreting IFN-γ, TNF-α, and IL-2 which are important for the protective effect against tuberculosis increased to a significant level.

The above-described description of the present invention is provided for illustrative purposes, and a person skilled in the art to which the present invention pertains will understand that the present invention can be easily modified into other specific forms without changing the technical spirit or essential features of the present invention. Therefore, it should be understood that the above-described embodiments are only illustrative in all aspects and not restrictive.

### [Industrial Applicability]

The glycosylated Ag85A protein according to the present invention can be usefully used as a vaccine for preventing tuberculosis. Furthermore, the glycosylated Ag85A protein can be effectively expressed in plants and separated with high yield by means of a vector optimized for protein production, and thus can be mass produced at low cost.

## Claims

1. A vaccine composition for preventing tuberculosis, comprising a glycosylated Ag85A protein comprising an amino acid sequence of SEQ ID NO: 1.

2. The vaccine composition of claim 1, wherein the glycosylated Ag85A protein induces inhibition of damage to lung tissue infected by *Mycobacterium tuberculosis.*

3. The vaccine composition of claim 1, wherein the glycosylated Ag85A protein induces a decrease in the number of *M. tuberculosis* in the lungs.

4. The vaccine composition of claim 1, wherein the glycosylated Ag85A protein induces an increase in CD4⁺CD44⁺ T cells simultaneously secreting two or more cytokines selected from the group consisting of IFN-γ (interferon-γ), TNF-α (tumor necrosis factor-α), and IL-2 (interleukin-2).

5. The vaccine composition of claim 1, wherein the glycosylated Ag85A protein induces an increase in CD8⁺CD44⁺ T cells simultaneously secreting two or more cytokines selected from the group consisting of IFN-γ (interferon-γ), TNF-α (tumor necrosis factor-α), and IL-2 (interleukin-2).

6. A vector for expressing a vaccine for preventing tuberculosis, comprising: a gene encoding a glycosylated Ag85A protein comprising an amino acid sequence of SEQ ID NO: 1; and a gene encoding cellulose binding module 3 (CBM3), comprising a base sequence of SEQ ID NO: 2.

7. A vector for expressing a vaccine for preventing tuberculosis, comprising: a gene encoding a glycosylated Ag85A protein comprising an amino acid sequence of SEQ ID NO: 1; a gene encoding cellulose binding module 3 (CBM3), comprising a base sequence of SEQ ID NO: 2; and a gene encoding a BiP signal peptide, comprising a base sequence of SEQ ID NO: 3.

8. A vector for expressing a vaccine for preventing tuberculosis, comprising: a gene encoding a glycosylated Ag85A protein comprising an amino acid sequence of SEQ ID NO: 1; a gene encoding cellulose binding module 3 (CBM3), comprising a base sequence of SEQ ID NO: 2; and a gene encoding an HDEL peptide, comprising a base sequence of SEQ ID NO: 4.

9. The vector of claim 6, wherein the vector further comprises a sequence which is recognized and cleaved by enterokinase, comprising a base sequence of SEQ ID NO: 5.

10. The vector of claim 6, wherein the vector further comprises a gene encoding a linking peptide, comprising a base sequence of SEQ ID: 6.

11. A transformant for preparing a vaccine for preventing tuberculosis, the transformant transformed with the vector of any one of claims 6 to 10.

12. The transformant of claim 11, wherein the transformant is a plant.

13. The transformant of claim 12, wherein the plant is one or more dicotyledonous plants selected from the group consisting of Arabidopsis thaliana, soybean, tobacco, eggplant, capsicum, potato, tomato, Chinese cabbage, radish, cabbage, lettuce, peach, pear, strawberry, water melon, Korean melon, cucumber, carrot, and celery; or one or more monocotyledonous plants selected from the group consisting of rice, barley, wheat, rye, corn, sugar cane, oat, and onion.

14. A method for producing a vaccine composition for preventing tuberculosis, the method comprising: (a) culturing the transformant of claim 11; and (b) isolating and purifying a glycosylated Ag85A protein from the transformant or culture solution.

15. A method for preventing tuberculosis, the method comprising: administering a vaccine composition comprising a glycosylated Ag85A protein comprising an amino acid sequence of SEQ ID NO: 1 to an individual.

16. A use of a vaccine composition comprising a glycosylated Ag85A protein comprising an amino acid sequence of SEQ ID NO: 1 for preventing tuberculosis.

17. A use of a glycosylated Ag85A protein comprising an amino acid sequence of SEQ ID NO: 1 for preparing a vaccine used for preventing tuberculosis.


<110>    BioApplications Inc.
         Industry-Academic Cooperation Foundation Yonsei University
         Quratis Inc.

<120>    Vaccine composition for preventing tuberculosis containing a
         glycosylated Ag85A protein and method for producing the vaccine
         composition

<130>    MPCT19-153

<150>    KR 10-2019-0010431
<151>    2019-01-28

<160>    8

<170>    KoPatentIn 3.0

<210>    1
<211>    301
<212>    PRT
<213>    Ag85A

<400>    1
Phe Ser Arg Pro Gly Leu Pro Val Glu Tyr Leu Gln Val Pro Ser Pro
  1               5                  10                  15

Ser Met Gly Arg Asp Ile Lys Val Gln Phe Gln Ser Gly Gly Ala Asn
             20                  25                  30

Ser Pro Ala Leu Tyr Leu Leu Asp Gly Leu Arg Ala Gln Asp Asp Phe
         35                  40                  45

Ser Gly Trp Asp Ile Asn Thr Pro Ala Phe Glu Trp Tyr Asp Gln Ser
     50                  55                  60

Gly Leu Ser Val Val Met Pro Val Gly Gly Gln Ser Ser Phe Tyr Ser
 65                  70                  75                  80

Asp Trp Tyr Gln Pro Ala Cys Gly Lys Ala Gly Cys Gln Thr Tyr Lys
                 85                  90                  95

Trp Glu Thr Phe Leu Thr Ser Glu Leu Pro Gly Trp Leu Gln Ala Asn
            100                 105                 110

Arg His Val Lys Pro Thr Gly Ser Ala Val Val Gly Leu Ser Met Ala
        115                 120                 125

Ala Ser Ser Ala Leu Thr Leu Ala Ile Tyr His Pro Gln Gln Phe Val
    130                 135                 140

Tyr Ala Gly Ala Met Ser Gly Leu Leu Asp Pro Ser Gln Ala Met Gly
145                 150                 155                 160

Pro Thr Leu Ile Gly Leu Ala Met Gly Asp Ala Gly Gly Tyr Lys Ala
                165                 170                 175

Ser Asp Met Trp Gly Pro Lys Glu Asp Pro Ala Trp Gln Arg Asn Asp
            180                 185                 190

Pro Leu Leu Asn Val Gly Lys Leu Ile Ala Asn Asn Thr Arg Val Trp
        195                 200                 205

Val Tyr Cys Gly Asn Gly Lys Pro Ser Asp Leu Gly Gly Asn Asn Leu

210                 215                 220

Pro Ala Lys Phe Leu Glu Gly Phe Val Arg Thr Ser Asn Ile Lys Phe
225                 230                 235                 240

Gln Asp Ala Tyr Asn Ala Gly Gly Gly His Asn Gly Val Phe Asp Phe
                245                 250                 255

Pro Asp Ser Gly Thr His Ser Trp Glu Tyr Trp Gly Ala Gln Leu Asn
            260                 265                 270

Ala Met Lys Pro Asp Leu Gln Arg Ala Leu Gly Ala Thr Pro Asn Thr
        275                 280                 285

Gly Pro Ala Pro Gln Gly Ala Gly Ser Gly Ser Gly Ser
    290                 295                 300

<210>    2
<211>    477
<212>    DNA
<213>    CBM3

<400>    2
gtatcaggta accttaaggt ggagttttac aactcgaacc cttctgatac aactaactca      60

ataaacccac agttcaaagt tacaaacaca ggcagctctg cgatcgattt gtctaaatta     120

accctcagat actattatac ggttgatgga cagaaggacc agactttctg gtgtgatcat     180

gcagctatca ttggttctaa cggtagctac aacggaatta catcaaacgt gaagggcact     240

ttcgttaaga tgtcctctag cactaacaac gcagacacat atttggagat cagttttacg     300

gggggaaccc ttgaaccagg tgctcacgtc cagattcaag gaagattcgc taaaaacgac     360

tggtcgaact atacccaaag taatgattac agttttaaat ccgcctcaca atttgttgag     420

tgggatcagg tcactgctta cctgaacggg gttctagtgt ggggaaagga acctggt        477

<210>    3
<211>    272
<212>    DNA
<213>    BiP

<400>    3
atggctcgct cgtttggagc taacagtacc gttgtgttgg cgatcatctt cttcggtgag      60

tgattttccg atcttcttct ccgatttaga tctcctctac attgttgctt aatctcagaa     120

ccttttttcg ttgttcctgg atctgaatgt gtttgtttgc aatttcacga tcttaaaagg     180

ttagatctcg attggtattg acgattggaa tctttacgat ttcaggatgt ttatttgcgt     240

tgtcctctgc aatagaagag gctacgaagt ta                                  272

<210>    4
<211>    12
<212>    DNA
<213>    HDEL

<400>    4
cacgatgagc tc                                                          12


<210>    5
<211>    15
<212>    DNA
<213>    nucleotide sequence recognized and cleaved by enterokinase

<400>    5
gatgacgacg ataaa                                                       15


<210>    6
<211>    33
<212>    DNA
<213>    linker peptide

<400>    6
gaggcagccg ctaaggaagc tgcagcgaaa gcc                                   33


<210>    7
<211>    903
<212>    DNA
<213>    Ag85A

<400>    7
tttagccggc ctggcctgcc tgtggaatac ctgcaggtgc ctagccctag catgggccgg      60

gacatcaaag tgcagtttca gagcggcggg gctaatagcc ctgctctgta cctgctcgat     120

ggcctgcggg ctcaggatga ttttagcggc tgggacatca acacccctgc ttttgaatgg     180

tacgatcaga gcggcctgag cgtcgtgatg cctgtgggcg ggcagagcag cttctacagc     240

gattggtatc agcctgcttg cggcaaagct ggctgccaga cctacaagtg ggaaaccttt     300

ctgaccagcg aactgcctgg ctggctgcag gctaatcggc acgtgaaacc taccggcagc     360

gccgtggtgg gcctgagcat ggctgctagc tccgctctga ccctggctat ctaccaccct     420

cagcagtttg tgtacgctgg cgctatgagc ggcctgctcg atccctccca ggctatgggc     480

cctaccctga tcgggctcgc tatgggggat gctggggggt acaaagctag cgatatgtgg      540

ggccctaaag aagatcctgc ttggcagcgg aatgatcctc tgctcaacgt gggcaaactg     600

atcgctaata atacccgagt gtgggtgtac tgcggcaatg gcaaacctag cgatctgggc     660

gggaacaatc tgcctgctaa atttctggaa ggcttcgtgc ggaccagcaa catcaagttt     720

caggatgctt acaatgctgg cgggggccac aatggcgtat ttgattttcc tgatagcggc     780

acccacagct gggaatactg gggcgctcag ctgaatgcta tgaaacctga tctgcagcgg      840

gctctgggcg ctacccctaa taccggccct gctcctcagg gcgctggctc cggatctggt     900

agt                                                                   903


<210>    8

<211>    159
<212>    PRT
<213>    CBM3

<400>    8
Val Ser Gly Asn Leu Lys Val Glu Phe Tyr Asn Ser Asn Pro Ser Asp
1               5                   10                  15

Thr Thr Asn Ser Ile Asn Pro Gln Phe Lys Val Thr Asn Thr Gly Ser
            20                  25                  30

Ser Ala Ile Asp Leu Ser Lys Leu Thr Leu Arg Tyr Tyr Tyr Thr Val
        35                  40                  45

Asp Gly Gln Lys Asp Gln Thr Phe Trp Cys Asp His Ala Ala Ile Ile
    50                  55                  60

Gly Ser Asn Gly Ser Tyr Asn Gly Ile Thr Ser Asn Val Lys Gly Thr
65                  70                  75                  80

Phe Val Lys Met Ser Ser Ser Thr Asn Asn Ala Asp Thr Tyr Leu Glu
                85                  90                  95

Ile Ser Phe Thr Gly Gly Thr Leu Glu Pro Gly Ala His Val Gln Ile
            100                 105                 110

Gln Gly Arg Phe Ala Lys Asn Asp Trp Ser Asn Tyr Thr Gln Ser Asn
        115                 120                 125

Asp Tyr Ser Phe Lys Ser Ala Ser Gln Phe Val Glu Trp Asp Gln Val
    130                 135                 140

Thr Ala Tyr Leu Asn Gly Val Leu Val Trp Gly Lys Glu Pro Gly
145                 150                 155